# EUROPEAN PATENT SPECIFICATION

(11) **EP 1 037 972 B1**
(45) Date of publication and mention of the grant of the patent: **14.09.2005**
(21) Application number: 98965565.9
(22) Date of filing: 18.12.1998
(51) Int. Cl.: C12N 15/10, C12N 15/85, C12N 15/86, C12N 9/00, C12N 7/01, C12N 5/10

(54) **NON-BACTERIAL CLONING IN DELIVERY AND EXPRESSION OF NUCLEIC ACIDS**
NICHT-BAKTERIELLES KLONIERUNGSSYSTEM ZUR VERABREICHUNG UND EXPRESSION VON NUKLEINSÄUREN
CLONAGE NON BACTERIEN POUR L'APPORT ET L'EXPRESSION D'ACIDES NUCLEIQUES

(30) Priority: 19.12.1997 US 995234
(43) Date of publication of application: 27.09.2000
(73) Proprietor: HK Pharmaceuticals, Inc., Massachusetts 02139 (US)
(72) Inventor: KECK, James, G., Redwood City, CA 94065 (US); MOLONY, Jocelyn, M., Castro Valley, CA 94552 (US); KUO, Sophia, S., San Francisco, CA 94131 (US)
(74) Representative: Baldock, Sharon Claire
(86) International application number: PCT/US1998/027942
(87) International publication number: WO 1999/032618

(56) References cited:
- WO-A-92/01786
- WO-A-94/20618
- WO-A-96/09392
- WO-A-98/32880
- WO-A-98/50530
- DE-C- 4 424 762
- GB-A- 2 319 773

## Description

### Technical Field

The present invention is related to methods and compositions for the high-throughput delivery and non-bacterial amplification of a nucleic acid of unknown function or oligonucleotides related to a nucleic acid of unknown function for identifying new genes or gene functions that are involved in specific cell phenotypes using catalytic RNA.

### Background

Many disease states are characterized by abnormal production or function of gene products such as proteins. The abnormality may be due to a defective gene, overproduction of a protein by a normal gene or improper temporal expression. Such abnormal or excessive production of protein can have direct effects on cells within the body or can initiate a cascade of events involving other proteins within the body, thereby producing undesirable effects. However, in many cases the disease-related gene is as yet unidentified.

Databases containing several million sequences of genes, gene fragments, and essential sequence tags (ESTs) have been developed and are continuously expanding; however, more economical and efficient methods are needed to determine the function(s) of the products encoded by these sequences and their role in disease or the expression of a given phenotype. Different approaches have been used to elucidate the function of an individual gene, including biochemical approaches, reverse genetics, genome characterization and sequencing, inactivation of gene function, gene expression and mutational analysis. Gene function can be inactivated at the DNA level by homologous recombination, by triplex technology in which triplex structures are formed which interfere with transcription of DNA into mRNA, by antibodies at the protein level or by antisense nucleic acids and ribozymes at the RNA level.

The development of transgenic techniques, in which a gene of interest has been inserted or deleted in a host cell, has been an important advance in the tools available for studying the function of genes at the organismal level. In particular, since the advent of automated DNA synthesis, oligonucleotides have been increasingly employed to analyze genes and their functions. Oligonucleotides expressed intracellularly in the form of polypeptides, antisense RNA and ribozymes have been used to alter gene expression as a method of determining and assigning gene function. However, these genetic approaches to elucidate gene function(s), as currently employed, share a common limitation, namely the need to identify, clone and sequence the gene of interest; the currently used techniques involve several bacterial or bacteriophage cloning and subcloning steps for the isolation, sequencing and manipulation of the gene of interest. This is a significant limitation, in terms of both time and expense. The requirement for multiple bacterial or bacteriophage cloning steps thus has rendered genetic techniques impractical for high-throughput screening assays for routine genetic analysis.

It therefore is of interest to develop a high-throughput system that avoids bacterial cloning for the delivery, expression and amplification of DNA and RNA that can be used to identify the function of a product encoded by a nucleic acid.

### Relevant literature

An RNA molecule not naturally occurring in nature having enzymatic activity independent of any protein is disclosed in USPN 4,987,071. General rules for the design of hammerhead ribozymes that cleave target RNA in *trans* are described in Haseloff and Gerlach (1988) *Nature* 334:585-591. Miniribozymes are disclosed in Uhlenbeck, (1987 *Nature* 328:596-603. Methods for optimizing cleavage of a target RNA by a ribozyme are described in USPN 5,496,698. Reporter gene suppression by engineered hammerhead ribozymes in mammalian cells is described in Cameron and Jennings, (1989) *Proc. Natl. Acad. Sci.* (USA) 86:9139-9143. Ribozyme expression from a retroviral vector is described in Sullenger and Cech, (1993) *Science* 262:1566-1569. The expression of hammerhead ribozymes operatively linked to a T7 promoter is described in Chowrira *et al*. (1994) *J. Biol. Chem.* 269:25856-25864. Co-localizing ribozymes with substrate RNAs to increase their efficacy as gene inhibitors is described in Sullenger, (1995) *Appl. Biochem. Biotechnol.* 54:57-61. Screening of retroviral cDNA expression libraries is described in Kitamura *et al*, (1995) *Proc. Nat. Acad. Sci. (USA)* 92:9146. Selection of efficient cleave sites in target RNAs by using a ribozyme expression library is described in Lieber and Strauss, (1996) *Mol. Cell. Biol.* 15:540-551. Approaches for the identification and cloning of differentially expressed genes is discussed in Soares, (1997) *Curr. Opin. Biotechnol.* 8:542-546. The development of high-throughput screen is discussed in Jayawickreme and Kost, (1997) *Curr. Opin. Biotechnol.* 8:629-634. The high-throughput screen for rarely transcribed differentially expressed genes is described in von Stein *et al*., (1997) *Nucleic Acids Res.* 25:2598-2602. High-throughput genotyping is disclosed in Hall, *et al*. (1996) *Genome Res* 6:781-790. The design of antisense RNA is disclosed in Sczakiel, (1997) *Antisense Nucleic Acid Drug Dev.* 7:439-444. Antisense mRNA regulation of gene expression is disclosed in USPN 5,457,281 USPN 5,190,931, and USPN 5,2720,65.

### SUMMARY OF THE INVENTION

Methods, and compositions for use therein, are provided for determining the function of a sample nucleic acid of unknown function, in which a non-bacterial cloning system is used for amplification of an oligonucleotide family which is related to the sample nucleic acid. The non-bacterial amplification method includes the steps of digesting an expression plasmid with two or more restriction enzymes so that the resulting digested expression vector is prevented from self-annealing and to provide directionality for the subsequent cloning of the nucleic acid-to-be-expressed which can be the sample nucleic acid or a related nucleic acid; simultaneously ligating in a matrix format the nucleic acid-to-be-expressed to the digested expression vector, so that one or more of the nucleic acids-to-be-expressed is operatively linked to transcriptional control elements in the digested expression vector; simultaneously introducing in a matrix format into a cell culture that contains one or more host cell(s) that express(es) a target nucleic acid(s) that contains the nucleotide sequence of the sample nucleic acid; and growing the cell culture so that the one or more of the nucleic acids-to-be-expressed are amplified and transcribed. The transcription product of the nucleic acid-to-be expressed is antisense RNA, expression of which is designed to alter the phenotype of the host cell. To identify and assign a function to the sample nucleotide sequence, the transfected or infected cell culture is analyzed for phenotypic changes produced as a result of the intracellular amplification and transcription of the expressed nucleic acid.

### BRIEF DESCRIPTION OF THE DRAWINGS

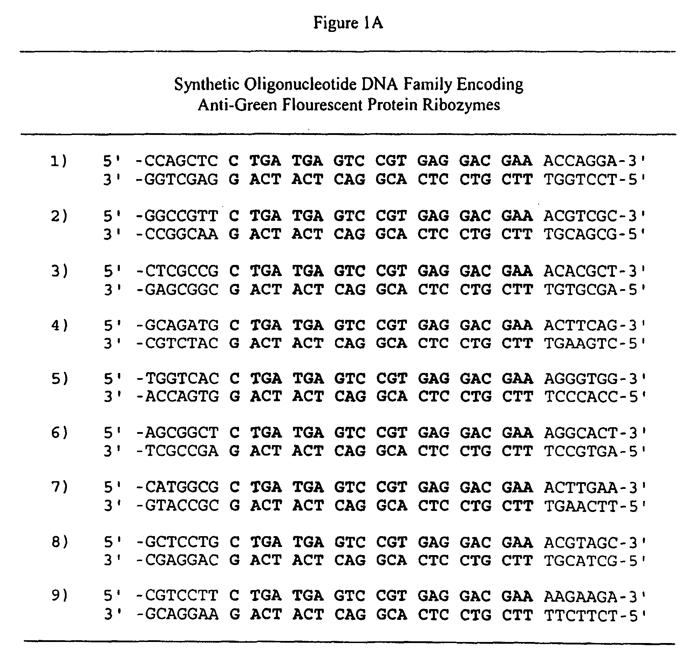

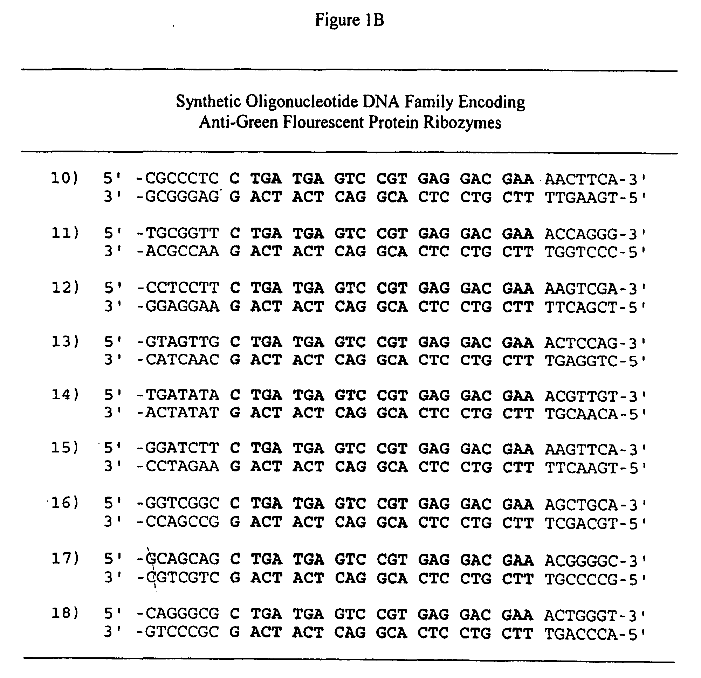

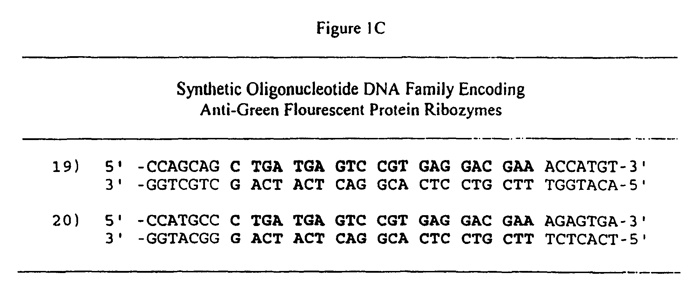

Figures 1A-1C show a family of oligonucleotides which encode ribozymes targeting the mRNA of green fluorescent protein (EGFP). The family of oligonucleotides was developed by identifying ribozyme consensus cleavage sites throughout the mRNA which are used as the basis for synthesizing DNA coding sequence of the complementary ribozyme sequences shown as the upper sequences in Figures 1A-1C (SEQ ID NOS:1-20). The lower sequences in Figures 1A-1C are the complementary sequences which are annealed to the ribozyme coding sequence to form double-stranded DNA for introduction into a host cell.

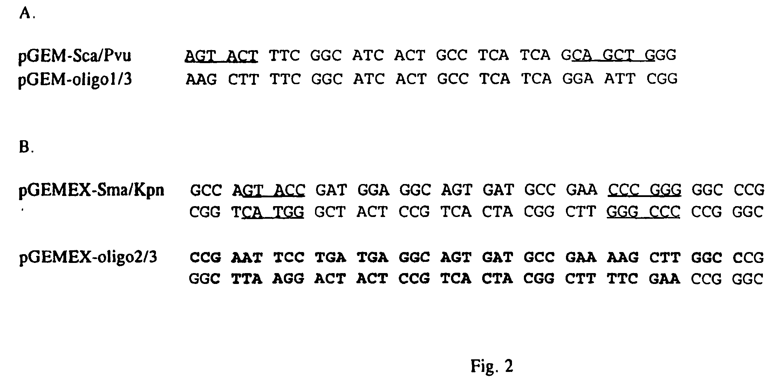

Figure 2 shows the sequences of delivery vectors containing incorporated oligonucleotides. A. shows the sequence of the insert region of pGEM-Sca/Pvu, (SEQ ID NO:38), obtained by sequence analysis and the predicted sequence of pGEM-oligo 1/3, (SEQ ID NO:39). Underlines represent the mutated sequences that were incorporated into the ribozyme vector. B. shows the sequence obtained for both pGEMEX-Sma/Kpn, (SEQ ID NO:40), and pGEMEX-oligo 2/3, (SEQ ID NO:41). Both strands of each ribozyme vector were sequenced. Underlines represent the mutated sequences that were incorporated into the ribozyme vector. Bold nucleotides represent the sequence original double-stranded oligonucleotide that was inserted into the vector.

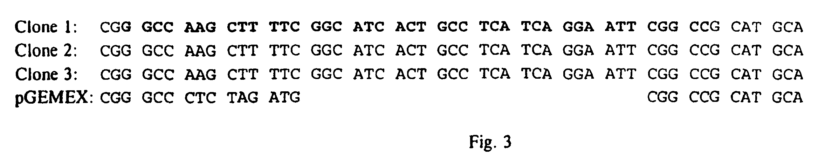

Figure 3 shows sequences of clones (pGEMEX-oligo2) containing an inserted oligonucleotide, (SEQ ID NO:42), compared with the sequence obtained from the parental plasmid, (SEQ ID NO:43). Bold nucleotides represent the sequence that is complementary to the sequence of the single-stranded oligonucleotide.

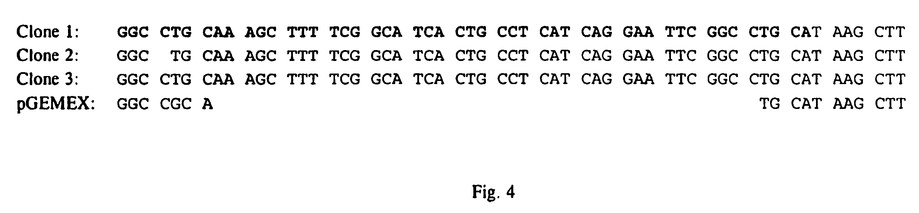

Figure 4 shows sequences of clones (pGEMEX-oligo1/5/6) containing an inserted oligonucleotide, (SEQ ID NOS:44-45), compared with the sequence obtained from the parental plasmid, (SEQ ID NO:46). Bold nucleotides represent the one full length strand formed from the triple-stranded oligonucleotide.

### BRIEF DESCRIPTION OF THE PREFERRED EMBODIMENTS

The present invention uses high-throughput assembly, delivery, amplification and expression of one or more nucleic acids from a plasmid or viral vector in a host cell culture which includes at least one host cell and can contain a plurality of host cells, as a means of assigning a function to a product of the host cell. The amplification of the expressed nucleic acids is performed in the host cell and does not require bacterial or bacteriophage cloning prior its introduction into the host cells. The nucleic acids-to-be-expressed are related to a sample nucleic acid of unknown function and code an antisense RNA, and include synthetic oligonucleotides, genomic DNA, cDNA, ESTs or fusion cDNA-ESTs. The sample nucleic acid is contained within the host cell by a target nucleic acid that codes for a product whose function is being assigned. The expression of the nucleic acid in the host cell alters the host cell's phenotype, thereby, assigning function to the product coded by the target nucleic acid. The term "function" is intended to mean a detectable or measurable event. The term "phenotype" is intended to mean a characteristic of a specific cell or cell population. Synthetic oligonucleotides are designed by analyzing the nucleotide sequence of the sample nucleic acid of unknown function and can be designed to encode an antisense RNA. Similarly, the antisense strand of the genomic DNA, cDNA or EST can be expressed. The nucleic acid-to-be-expressed is related to a sample nucleic acid sequence of unknown function which can be genomic DNA, cDNA, EST or RNA or a nucleotide sequence derived or obtainable from any of the above.

By "polypeptide" is intended a molecule containing two or more amino acids joined by peptide bonds. By "fusion polypeptide" is intended a polypeptide attached to a label or tag. By "antisense RNA" is intended an RNA molecule that binds to a target nucleic acid molecule, thereby, inhibiting its function and the expression of the encoded product. By "ribozyme" is intended an RNA molecule that acts as an enzyme and has been engineered to cleave other RNA molecules; after binding selectively to its specific RNA target, it acts catalytically to cut, or cleave, the target RNA molecule.

The nucleic acids-to-be-expressed are introduced into any of a variety of vectors, depending on the availability of restriction enzyme sites, intracellular location, and transcriptional regulatory elements for delivery and expression of the nucleic acid in the target host cell. When the nucleic acids-to-be-expressed are oligonucleotides, they can be simultaneously synthesized on solid supports in a matrix format, and simultaneously deprotected and cleaved. If complementary pairs of oligonucleotides are simultaneously synthesized, deprotected and cleaved in a matrix format, they can be simultaneously annealed and ligated to a vector. Alternatively, a single oligonucleotide is annealed to the delivery vector, ligated and the complementary strand can be filled in by a DNA polymerase or the complementary strand can be filled in before ligation. Three oligonucleotides can be annealed together with the delivery vector; intervening gaps are filled in by a DNA polymerase and ligated with a DNA ligase. The delivery vector containing the nucleic acid-to-be-expressed optionally is treated to increase the supercoiling of the delivery vector DNA, for example using DNA gyrase so as to improve uptake of the DNA into a recipient cell, such as a packaging cell or the intended target host cell. From one to multiple nucleic acids in a chosen delivery vector are introduced into the recipient cell. When a retrovirus vector is used, following preparation of recombinant retrovirus in a packaging cell, the recombinant retrovirus is used to infect a host cell containing the target nucleic acid. Instead of using a viral vector to make a virus for infection, a plasmid expression vector can be transfected directly into a host cell containing the nucleic acid of interest. The infected or transfected host cell is grown and the phenotype of the infected or transfected host cell is analyzed to determine any alterations in phenotype as compared to an uninfected or untransfected host cell. Alterations in cell phenotype are then correlated with the target nucleic acid so as to assign a function to a product coded for by the sample nucleic acid.

There are several advantages to the subject invention. The ability to eliminate the amplification step of plasmid DNA in bacteria such as *E. coli* is a major cost saving step as well as a time saving step over existing technologies: *E. coli* amplification can add several days onto the entire process. The subject process lends itself to automation when implemented in a 96-well or similar multi-well or matrix format. In addition, the expression, antisense RNA of the disclosed invention offer several advantages over currently available techniques. High-throughput delivery and expression of these molecules from a gene vector family offers the advantage that multiple members of these molecules can be introduced into and expressed in host cell cultures to enable identification of genes by a manageable screening process. The number of antisense RNA's expressed per cell depends on the multiplicity of infection for a virus vector or the amount of DNA transfected per cell for a plasmid vector. The high-throughput delivery permits the screening of a large number of nucleic acid constructs in a relatively short period of time. Furthermore, the construction of the antisense RNA with synthetic oligonucleotide DNA offers the additional advantage that directionality is conveniently achieved by incorporating unique restriction enzyme sites at both ends of each of the oligonucleotides used to prepare the double-stranded DNA coding for these molecules so that double-stranded DNA is ligated to the delivery vector in the correct orientation for expression. If the same restriction enzyme sites, blunt ends or restriction enzyme sites comprising compatible cohesive ends are used for the ligation, theoretically about 50% of all the constructs would be expressed in the incorrect orientation.

Other advantages of the subject invention include the capability to regulate the magnitude and timing of nucleic acid expression and high-throughput delivery. Operatively linking the nucleic acids-to-be-expressed to a regulatable promoter provides temporal and/or cell type specific control throughout the screening assay. Additionally, the magnitude of expression can be modulated using promoters that differ in their transcriptional activity.

Oligonucleotide DNA can be designed to express antisense RNAs. The strand coding the antisense RNA and the complementary strand are prepared individually and annealed to form double-stranded DNA which may include a means for determining directionality of expression. The double-stranded DNA is formed either by annealing two complementary oligonucleotides to form a double-stranded oligonucleotide or by annealing three oligonucleotides to form a triple-stranded oligonucleotide that contains single-stranded gaps and single stranded ends that are complementary to the ends of a delivery vector. Single-stranded portions of the oligonucleotide/vector can be filled in by treatment with a DNA polymerase such as Klenow, Taq polymerase or a cellular polymerase. Single-stranded ends of the triple-stranded oligonucleotide contain excess nucleotides that may be of any length but are preferably between two and ten nucleotides and most preferably between three and five nuelcotides. In addition, genomic DNA, cDNA or an EST can be directly expressed as an antisense mRNA.

In the following examples, a ribozyme family is targeted to a reporter gene, green fluorescent protein (GFP), that is expressed in CHO-AA8 Tet-Off cells or can be expressed in 293 Tet-Off cells (Clontech, Palo Alto, CA). GFP from the firefly *Aequorea victoria* emits bright green light upon mere exposure to UV or blue light and unlike other bioluminescent reporters, does not require additional proteins, substrates, or cofactors to emit light. EGFP encodes a protein which has a single, red shifted spectrum and increased expression relative to GFP, and therefore, is easily monitored in living cells by fluorescence microscopy and fluorescence-activated cell sorting (FACS). Ribozyme activity is inversely proportional to reporter gene signal. Other reporter genes, for example chloramphenicol acetyltransferase (CAT), beta-galactosidase, or alkaline phosphatase, also can be used.

For annealing the complementary oligonucleotide DNA strands, special conditions are not required. For example, both strands can be dissolved in water then mixed at a one to one molar ratio. They can be mixed in almost any buffer system, T4 ligase buffer, Exonuclease 3 buffer, Mung Bean Nuclease buffer. No special heating is required, room temperature is adequate. This mixing and annealing of the oligonucleotide strands generally occurs in a 96-well microtiter dish although other appropriate apparatus also can be used.

The means for determining directionality of expression conveniently is the use of unique non-blunt end forming restriction enzyme sites at both ends of the oligonucleotide DNA, thus the two oligonucleotides to be annealed share complementary sequences except at the ends where they will be able to bind to a restriction enzyme site. For the restriction enzyme sites, any non-blunt end forming restriction enzyme site can be used at either end, depending on the sites within the DNA vector into which the oligonucleotide DNA fragment is to be ligated. Having different sites at each end gives directionality. Any restriction enzyme that produces unique non-blunt cohesive ends suitable for ligation by DNA ligase can be used, for example *Aat* II, *Eco*R I, *Bam*H I, *Hind* III, *Pst* I. If necessary, a restriction enzyme site with a cohesive end can be used with a restriction enzyme site that produces a blunt end. Alternatively, the two oligonucleotides can be completely complementary, including the ends and digested with restriction enzymes prior to ligation with the delivery vector. In this case, it is preferred that the restriction enzyme sites does not occur within the oligonucleotide DNA coding region, otherwise partial digestions will be required.

The delivery vector into which the nucleic acids-to-be-expressed are to be ligated is digested with the appropriate restriction enzymes, either simultaneously or sequentially, to produce the appropriate ends for directional cloning of the nucleic acid whether it be oligonucleotides, genomic DNA, cDNA, or a previously cloned DNA. The nucleic acids-to-be-expressed preferably contain compatible ends to facilitate ligation to the vector in the correct orientation. For the example of oligonucleotide ligation, the ends compatible to the vector can be designed into the oligonucleotides. When the nucleic acids-to-be-expressed are genomic DNA, cDNA, or a previously cloned DNA, the compatible ends can be formed by restriction enzyme digestion or the ligation of linkers to the DNA containing the appropriate restriction enzyme sites. Alternatively, the vector can be modified by the use of linkers. The restriction enzyme sites are chosen so that transcription of the nucleic acid from the vector produces an antisense RNA targeted to the mRNA of interest. Restriction enzyme digestion is routinely performed using commercially available reagents according to the manufacturer's recommendations and will vary according to the restriction enzymes chosen. Once digested, the vector and nucleic acids-to-be-expressed can be purified by gel electrophoresis, chromatography or phenol/chloroform extraction and ethanol precipitation. The optimal purification method depends on the size and type of the vector and nucleic acids. Both can be used without purification. Generally, the nucleic acids-to-be expressed contain 5'-phosphate groups and the vector is treated with alkaline phosphatase to promote nucleic acid-vector ligation and prevent vector-vector ligation. If the nucleic acids-to-be-expressed are synthetic oligonucleotides, 5'-phosphate groups are added by chemical or enzymatic means before annealing. For ligation, ratios of nucleic acids-to-be-expressed DNA to vector DNA range from approximately 4:1 to 6:1. The ligation reaction is performed using T4 DNA ligase or other enzyme that catalyzes double-stranded DNA ligation. Reaction temperature and time can vary from 15°C to room temperature and from 18 hours to 5 minutes, respectively.

Delivery vectors produced by any of the above-identified methods can be amplified by transfecting cells (prokaryotic or eukaryotic) with the ligation mixtures and selecting for cells that contain the recombinant vectors. The selection method can employ drug resistance markers, visual markers such as green fluorescent protein or other selectable markers. Subsequent amplification of cells followed by isolation of vector DNA results in the production of novel ribozyme vectors.

One method for expression of the nucleic acids employs recombinant retroviruses. These vectors generally include as operatively linked components, retroviral long terminal repeats, packaging sequences and cloning site(s) for insertion of heterologous sequences. Other operatively linked components may include a nonretroviral promoter/enhancer and a selectable marker gene. Examples of retrovirus expression vectors which can be used include DC-T5T (Sullenger *et al*. 1990. *Mol. Cell Biol.* 10:6512-65230), *kat* (*Blood.* 1994 83:43-50), BOSC (*Proc. Natl. Acad. Sci. (USA)* (1993) 90:8392-8396), pBabe (*Proc. Natl. Acad. Sci. (USA)* (1995) 92:9146-9150) and RetroXpress™ (Clontech, Palo Alto, CA).

In some instances, it is desirable to increase expression of the designed oligonucleotides utilizing other promoters and/or enhancers in place of the promoter and/or enhancers provided in the expression vector. These promoters in combination with enhancers can be constitutive or regulatable. Any promoter/enhancer system functional in the target cell can be used. (*See* for example, *Molecular Virology* pp. 176-177; Hofmann, *et al*. 1996. *Proc. Natl. Acad. Sci. (USA)* 93:5185-5190; Coffin and Varmus, 1996. Retroviruses. Cold Spring Harbor Press. NY; Ausubel *et al*. 1994. Current Protocols in Molecular Biology. Greene Publishing Associates, Inc. & Wiley and Sons, Inc.). Examples include: CMV immediate-early promoter, SV40, thymidine kinase promoter, metalothionine promoter, and tetracycline operator (Hofman *et al*., (1996) *Proc. Natl. Acad. Sci (USA)* 93:5185-5190).

To package the recombinant retrovirus vectors containing the nucleic acid-to-be-expressed, cells lines are used that provide in *trans* the gene functions deleted from the recombinant retrovirus vector such that the vector is replicated and packaged into virus particles. The genes expressed in *trans* encode viral structural proteins and enzymes for packaging the vector and carrying out essential functions required for the vector's expression following infection of the target host cell. Packaging cell lines and retrovirus vector combinations that minimize homologous recombination between the vector and the genes expressed in *trans* are preferred to avoid the generation of replication competent retrovirus. Packaging systems that provide essential gene functions in *trans* from co-transfected expression vectors can be used and packaging systems that produce replication competent retroviruses. Following packaging, the recombinant retrovirus is used to infect target cells of interest. The envelope proteins expressed should permit infection of the target cell by the recombinant retrovirus particle. Retrovirus packaging cell lines which can be used include BOSC23 (*Proc. Natl. Acad. Sci. (USA)* 90:8392-8396), PT67 (Miller and Miller. 1994. *J. Virol*. 68:8270-8276, Miller. 1996. *Proc. Natl. Acad. Sci. (USA)* 93:11407-11413), PA317 (*Mol. Cell Biol.* 6:2895 (1986)) and PG13.

Other methods to obtain recombinant retrovirus particles also may be used. For example, the nucleic acids-to-be-expressed are functionally linked to eukaryotic transcriptional elements and is flanked by a retroviral packaging signal and 5' and 3' LTRs. This entire retrovirus construct is functionally linked to the T7 RNA polymerase promoter and T7 terminator. Also encoded by the vector but not within the retroviral construct is a gene functionally linked to a eukaryotic promoter that expresses a T7 RNA polymerase (T7pol) that contains a nuclear localization signal (T7pol-nls). Following transfection of this vector into a retroviral packaging cell, the T7-nls is expressed and localized in the nucleus where it transcribes recombinant retroviral genomes that are packaged by the retroviral genes expressed by the packaging cell. Because of the high transcriptional activity of T7pol-nls, high titered recombinant retrovirus titers can be achieved. Similar vectors, utilizing other DNA-dependent RNA polymerases, such as, SP6 or T3 also can be used.

In addition to recombinant retrovirus systems, other viral packaging systems such as adenovirus-associated virus (AAV), adenovirus, Sindbis virus, Semliki Forest virus, Epstein Barr virus, herpes simplex virus, HIV, or vaccinia virus. Each of these systems has a different host range and can be used to infect cells that are refractory to retrovirus expression, i.e., non-dividing cells. In the Sindbis virus system (Invitrogen, San Diego, CA), the nucleic acids-to-be-expressed are ligated into the multiple cloning site of a Sindbis virus DNA vector, i.e., pSinRep5, operatively linked to the Sindbis subgenomic promoter and polyadenylation site; the nucleic acids-to-be-expressed replaces the Sindbis virus structural protein genes. pSinRep5 includes an SP6 RNA polymerase promoter for the *in vitro* synthesis of recombinant Sindbis virus genomes; a packaging signal for recombinant RNA packaging; and the Sindbis nonstructural polyprotein gene open reading frame. For the production of Sindbis virus particles, the recombinant Sindbis vector encoding the oligonucleotide DNA is linearized, transcribed into RNA and co-transfected into vertebrate (BHK-21, Vero) or invertebrate cells (*Drosophila*) with RNA transcribed from the helper vector, pDH-BB, that encodes the viral structural proteins. Following transfection, the recombinant Sindbis genomic RNA acts as a mRNA, is translated into the Sindbis virus polymerase, and expresses the encoded polypeptide, fusion polypeptide, antisense RNA or ribozyme from the subgenomic promoter and the structural proteins from the helper RNA. Because of Sindbis virus' host range, the recombinant Sindbis virus can be packaged and used to express the encoded polypeptide, fusion polypeptide, antisense RNA or ribozyme in mammalian, avian, reptilian, mosquito and *Drosophila* cells (*see* for example, Xong, C. *et al*. (1989) *Science* 243:1188-1191; Huang, H.V. *et al*. (1993) United States Patent Number 5,217,879; Hahn C.S. *et al*. (1992) *Proc. Natl. Acad. Sci.* (USA) 89:2679-2683; Huang, M. and Sommers, J. (1991) *J. Virol.* 65:5435-5439).

For expression in AAV, the nucleic acid is cloned into an AAV expression vector, such as ALAPSN, that contains a cloning site functionally linked to a Moloney leukemia virus promoter and flanked by AAV terminal repeats and a packaging signal. ALAPSN also comprises a neomycin resistance gene functionally linked to SV40 transcription control elements. Similar AAV vectors, such as CWRSP and CWRSP.N, with comparable features may also be used. To produce recombinant AAV particles, 293 cells are infected with adenovirus type 5; 4 hours later the infected cells are co-transfected with the ALAPSN plasmid-oligonucleotide DNA construct and an AAV helper plasmid, pAAV/Ad (Samulski *et al*., (1989) *J Virol.* 63:3822-3828). As recombinant AAV is produced, the 293 cells undergo cytopathology, becoming spherical and lose their ability to adhere to a tissue culture surface. Following development of maximal cytopathology the supernatant is harvested and, if necessary, concentrated (Halbert *et al*. 1997. *J. Virol.* 71:5932-5941).

For vaccinia virus expression, a replication competent vaccinia virus can be used. The nucleic acids-to-be-expressed are operatively linked to a vaccinia virus promoter, for example, P11. In a preferred embodiment, vaccinia virus strain MVA is used because it expresses recombinant genes but contains a deletion that renders it replication incompetent in many mammalian cells. Therefore, the nucleic acids can be expressed in target host mammalian cells without the development of vaccinia virus induced cytopathology. The recombinant vaccinia virus strain MVA is produced by infecting chicken embryo fibroblasts (CEF) with vaccinia MVA and transfecting the transfer vector, pG01, into which has been ligated the ribozyme and a marker gene (beta galactosidase) functionally linked to a vaccinia promoter, such as P11, and flanked by the MVA genome sequences that flank the site of the MVA genomic deletion. The P11-ribozyme/beta-galactosidase construct is inserted into the MVA genome by homologous recombination. Recombinant viruses can be identified by *in situ* staining for beta-galactosidase expression with X-gal (Wyatt *et al*. (1995) *Virology* 210:202-205).

The nucleic acids can also be expressed from plasmid expression vectors that are transfected directly into mammalian cells. The direct delivery of the plasmid expression vector into the mammalian cell without an intervening bacterial cloning or transformation step provides a significant savings in time and expense and increases the number of sample nucleic acids that can be studied. Expression plasmids containing cloning sites operatively linked to either SV40, CMV, metallothionine, or tetracycline transcriptional regulatory elements can be used: pCEP4 (Invitrogen, San Diego, CA), pCMVβ, (Clontech, Palo Alto, CA), pAlter®-MAX (Promega, Madison, WI). The plasmid preferably contains sequences to provide high-copy episomal replication and selectable markers for stable maintenance of the vector in the host cell. The plasmids containing the nucleic acids-to-be-expressed are transfected directly into the target cell of interest. In a preferred embodiment, the plasmids are supercoiled with a gyrase to increase transfection efficiency. In another embodiment, the nucleic acids-to-be-expressed are ligated into plasmids and functionally linked to the T7, SP6, T3 or a similar RNA polymerase promoter. The plasmid expression vectors that can be used include: pGEM-3Z, pAlter®-*Ex*1 (Promega, Madison, WI). The plasmid-nucleic acids-to-be-expressed construct is transfected into mammalian cells that are infected with vaccinia strain MVA that expresses the appropriate RNA polymerase (Wyatt *et al*., (1995) *Virology* 210:202-205). For the example of vaccinia MVA T7, the T7pol transcribes the nucleic acids-to-be-expressed from the plasmid vector. The vaccinia MVA amplifies the plasmid-nucleic acid construct resulting in an increased intracellular template concentration for T7pol transcomplementation and increased antisense RNA expression.

Other systems for the expression of nucleic acids functionally linked to T7 RNA polymerase or other bacteriophage promoters (SP6 or T3) may also be used. Nucleic acid expression can be performed with a recombinant retrovirus vector containing the nucleic acid-to-be-expressed functionally linked to a T7 RNA polymerase promoter (T7pro) and T7 terminator. This expression cassette is flanked by 5' and 3' LTRs, a packaging signal and includes the T7pol gene, that encodes a T7pol that contains a nuclear localization signal (T7pol-nls), functionally linked to a eukaryotic promoter. In this system, the expressed T7 protein is transported to the nucleus for nucleic acid transcription. Because of the high transcriptional activity of T7pol, high intracellular levels of polypeptide, fusion polypeptide, antisense RNA or ribozyme can be achieved. In another embodiment, antisense RNA can be fused to another ribozyme that acts intramolecularly to free the antisense RNA targeting the nucleic acid of interest.

Transfection of nucleic acid (DNA or RNA) into cells is required for either packaging of recombinant vectors into virus particles or direct transfection of plasmids that express antisense RNA, into target host cells can be mediated by a variety of chemicals including calcium-phosphate, polybrene, DEAE-dextran, and liposomes. The calcium-phosphate method includes contacting the surface of the cell with a calcium phosphate-DNA co-precipitate. Polycations such as polybrene or DOSPER (Boehringer-Mannheim) also can be used to increase the efficiency of transfection of low molecular weight DNA. Liposomes are available from a variety of commercial suppliers and include DOTAP™ (Boehringer-Mannheim), Tfx™-50, Transfectam®, ProFection™ (Promega, Madison WI), and LipofectAmin™, Lipofectin®, LipofectAce™ (GibcoBRL, Gaithersburg, MD). In solution, the lipids form vesicles that associate with the nucleic acid and facilitate its transfer into cells by fusion of the vesicles with cell membranes or by endocytosis. Alternatively, DNA can be introduced into cells by electroporation. Each of these systems differ in their transfection efficiency for a given cell line. If transfection conditions for a given cell line have not been established or are unknown, they can be determined empirically (Maniatis *supra*).

The target host cell can be any cell of interest that expresses a disease associated phenotype or a phenotype that can be differentiated from a "normal" or control cell. For example, the host cell can be a mammalian cell, a plant cell or a microorganism. If a tumor cell expresses genes, for example, H-*ras*, that are not expressed in normal cells, antisense RNA targeting these genes are constructed and expressed in the tumor cells that are then assayed for an altered cell phenotype. The altered phenotype may be reduced cell growth, reduced DNA synthesis, increased synthesis of a protein(s), chemical responsiveness, morphologic changes. Tumor cells have been shown to differentially express between 138-500 genes compared to their non-tumorigenic counterparts. Examples of tumor cells are EJ (bladder carcinoma), FEM (melanoma), RT-4 (bladder carcinoma), MCF-7 (breast carcinoma), PC3 (prostate carcinoma). Examples of genes expressed in tumor cells are *fos*, H-*ras* or the estrogen receptor. Proteins that regulate gene expression in tumor cells also can be identified. For example, this can be accomplished by monitoring the expression of a reporter gene expressed from a promoter that is active in tumor cells in the presence of antisense RNA targeted to a gene of interest. Conversely, for gene(s) that are expressed in particular normal cells but are not expressed in tumor cells, antisense RNA can be expressed in the normal cells which are then assayed for an altered phenotype. In either approach, gene(s) associated with disease pathways or phenotypes can be identified.

For determining the function of the gene of interest, the tumor or target cells are evaluated for an alteration in their phenotype. Examples of phenotypic changes that can be evaluated include cell viability, replication, morphologic changes, membrane permeability, protein expression or the expression of biologically active compounds (e.g., steroids), proliferation, drug susceptibility, expression of cell surface molecules such as receptor molecules and antigens. In order to evaluate an alteration in cell phenotype, any of a variety of methods can be used, depending at least in part on the amount of information available concerning the putative function of a product encoded by a target nucleic acid.

In host cells amplifying and expressing antisense RNA, phenotypic change can be monitored directly. For example, if a product of the target nucleic acid prevents apoptosis is inhibited by an antisense RNA, the host cell will undergo specific types of morphologic changes, such as nuclear condensation, following oligonucleotide DNA expression. The function of cellular or viral genes also can be identified that are involved in virus replication. Antisense RNA can be designed to viral genes or cellular genes whose expression is altered by virus infection. If the product of the targeted nucleic acid affects replication, virus titers may increase or decrease. If the product of the nucleic acid is involved in drug susceptibility, this function can be identified by monitoring cells for altered resistance or susceptibility to various drugs.

The observed changes a cell undergoes as a result of oligonucleotide DNA expression can be monitored by various methods. Stains can be employed if the decreased expression of a targeted gene affects cell viability or membrane permeability. The expression of a reporter gene operatively linked to a promoter that is regulated by the product of the targeted nucleic acid can also be used. If the product of the target nucleic acid affects cell cycle regulation and transformation this can be monitored by measuring the incorporation of a labeled nucleotide into the cell. Antibody-based assays can be employed to detect the presence or absence of a protein of interest coded for by a nucleic acid of interest. Additional types of assays can be employed depending on the phenotype or cellular property that is being analyzed.

Phenotypic change can be monitored indirectly, for example, by evaluating ribozyme activity by comparing the targeted mRNA levels in cells expressing and cells that do not express one or more members of the ribozyme family designed to inhibit the function of the targeted mRNA and its encoded product. Total cellular or cytoplasmic RNA can be purified by a variety of methods (Maniatis *supra* pp. 7.6-7.29) and analyzed by Northern or dot blot (Maniatis *supra* pp. 7.37-7.57). mRNA can be assayed by reverse transcription-PCR employing primers that hybridize with the targeted mRNA. The absence or decreased production of a PCR product is indirectly indicative of ribozyme activity (Baier *et al.* 1994. *Molecular Immunology* 31:923-932). Similar methods can be used to evaluate antisense RNA expression. Because antisense RNA does not act catalytically to cleave its target mRNA, antisense expression can be monitored by treating the purified RNA with an nuclease specific for double-stranded RNA followed by Northern blot, dot blot or reverse transcription-PCR. To monitor the expression of a polypeptide or fusion polypeptide, protein electrophoresis, antibody-based assays, Northern blots, and reverse-transcription PCR can be utilized to detect either the polypeptide or fusion polypeptide directly or the encoding mRNA.

The methods and compositions of the subject invention can be used to identify the function of genes. The function of genes can be identified that are involved in virus replication or life cycle. If the product of the targeted nucleic acid affects replication, virus titers may increase or decrease. In this example the target gene can be a cellular or a viral gene. If the targeted nucleic is in a bacteria or microorganisms, determining their function identifies new pathways and lead to the identification of targets for new antimicrobials.

The following examples are offered by way of illustration and not by way of limitation.

### EXAMPLES

### Example 1

### Ribozyme Design

The purpose of this experiment was to design a oligonucleotide family encoding ribozymes that would cleave the EGFP mRNA (Clontech, Palo Alto, CA) (SEQ.ID NO. 1). Twenty ribozymes were prepared to target at approximately equidistant points in the GFP mRNA sequence from a putative GUN (N=A, G, C, U) optimum cleavage site (Kashani-Sabet and Scanlon, (1995) *Cancer Gene Therapy* 2:213 -223).

An oligonucleotide family for ribozyme expression, Figure 1A-1C, were made in a 96-well matrix using parallel array technology and annealed to form double-stranded DNA with unique *Hin*d III and *Cla* I sites at each of the 5' and 3' ends, respectively, for ligation into a retrovirus vector. An example of two complementary oligos is as follows, with the catalytic core from a hammerhead ribozyme in bold. The underlined region is the ribozyme binding sequence when expressed as RNA that is complementary to a mRNA sequence of EGFP.

To anneal the complementary oligonucleotides, approximately 1.0 microgram of each set shown in Table 1 were dissolved in water and mixed at a one to one molar ratio in a 96-well microtiter plate at room temperature. The 5' end (left end) of the double-stranded DNA fragment overlaps with an *Hin*d III restriction enzyme site. The 3' end of the fragment (right end) overlaps with a *Cla* I site.

### Example 2

### Preparation of a Family of Retrovirus Plasmid Vectors

The purpose of this experiment was to prepare a family of retrovirus plasmid vectors comprising a double-stranded DNA encoding a ribozyme to EGFP mRNA. pLNCX, (50 micrograms, Clontech, Palo Alto, CA), which contains an extended viral packaging signal, multiple cloning site and neomycin resistance gene flanked by the Moloney murine leukemia virus 5' and 3' long terminal repeats and an ampicillin resistance gene was digested with restriction enzymes, *Hin*d *III* and *Cla* I. Approximately, 0.5 to 2 µg of digested plasmid was placed into a well of a multi-well (e.g. 96 well) plate. The family of annealed oligonucleotides, were added individually at 4 to 6 fold excess of the *Hin*d III/*Cla* I treated pLNCX. The oligonucleotide DNA was ligated into pLNCX by adding a tenth volume of 10X T4 DNA ligation buffer and T4 DNA ligase. The final concentration of the ligation buffer components and T4 DNA ligase were: 0.05 M Tris-HCl (pH 7.6), 10 mM MgCl₂, 10 mM dithiothreitol (DTT), 50 µg/ml bovine serum albumin (Fraction V; optional), 1.0 mM ATP, 0.05 Weiss units of bacteriophage T4 DNA ligase per microliter. The ligation was performed for 4-8 hours at 16°C.

### Example 3

### Transfection of Mammalian Cells Using Retrovirus Plasmid Vector

The purpose of this experiment was to package the family of retrovirus virus plasmid constructs from Example 2 into retrovirus virus particles. Using the Calcium phosphate precipitation method (Keck, *et al*. (1990) *Cell* 61:801-809, Cochran, *et al*. (1985) *Proc. Natl. Acad. Sci. (USA)* 82:19-23) for transfecting DNA into mammalian cells, 0.1 to 0.5 micrograms of ligated plasmid/oligo DNAs from Example 2 was transfected per well of a 96-well plate containing 100 µl of minimal essential media (MEM) supplemented with 10% fetal calf serum (FCS) and approximately 1,000 to 25,000 PT67 cells. Four hours later the media was replaced with fresh MEM supplemented with 10% FCS and incubated at 37°C for 48 hours. The retrovirus vector contains a neomycin resistance gene; therefore, G418 selection can be used to obtain a population of cells that stably express the transfected vector and to monitor virus titers. Recombinant retrovirus production can be monitored by titering aliquots of the transfected cell supernatant in a focus forming assay whereby cells infected with the recombinant retrovirus become resistant to G418 (Clontech, Palo Alto, CA). When virus titers >10⁵/ml were reached, usually between 2-7 days, the viruses are infected into target cells, CHO-AA8- Tet-Off cells (Clontech, Palo Alto, CA), expressing EGFP (CHO-EGFP).

### Example 4

### Analysis of Ribozyme Activity

This experiment was designed to demonstrate the inactivation of EGFP expression in target host cells infected with a family of recombinant retrovirus vectors that express ribozymes targeted to various regions of the EGFP mRNA. Ribozyme activity is inversely proportional to EGFP expression. The ribozyme hybridizes to and cleaves the EGFP mRNA, thereby, reducing protein expression. EGFP was assayed fluorescence by an automated Fluorescence Activated Cell Sorter.

CHO-EGFP cells (Clontech, Palo Alto, CA) were cultured to near confluency in 96-well plates in MEM with 10% FCS. The family of recombinant retroviruses from Example 3 were used to individually infect (one recombinant retrovirus/well) CHO-EGFP cells. The multiplicity of infection (virus particle per cell ratio) was about 5-10 to insure that every cell was infected with at least one virus particle. Infection of target cells was enhanced with polybrene (10 micrograms/ml), a polycation that acts by neutralizing the net negative surface charges on the virus and cells (Stoker. "Retroviral Vectors" In Molecular Virology: A Practical Approach, Davison and Elliott, eds., p 187). Mock infected cells served as controls. Following infection, the cells were incubated for 48 hours at 37°C and then assayed for EGFP expression.

EGFP expression was assayed by using an incident light at 488 nm and measuring the emitted light at 507 nm. The emitted or observed light is detected in using the appropriate set of filters, corresponding to the incident and emitted light and a Wallac-Victor Machine or by a Floursence Activated Cell Sorter (FACS).

### Example 5

### Preparation of Plasmid Vector for Non-Retroviral Transfection

This example discloses the construction of plasmid vectors that express the family of ribozymes without the need for a bacterial cloning step. The family of EGFP ribozymes were ligated into the multiple cloning site of pCEP4 (Invitrogen, San Diego, CA) operatively linked to the CMV promoter and SV40 polyadenylation signal. pCEP4 is an Epstein Barr virus (EBV)-based vector that is maintained extrachromosomally in primate and canine cell lines. pCEP4 contains nuclear antigen, EBNA-1, for high-copy episomal replication of the plasmid by the EBV origin of replication, *ori*P, and the hygromycin resistance gene for stable maintenance of the vector. In this example, the oligonucleotides are designed to contain *Hin*d III and *Bam*H I sites to facilitate ligation into the expression vector. The synthesis, annealing and ligation procedures are the same as those described for the retrovirus vectors in Example 1.

### Example 6

### Transfection of Mammalian Cells (Non-Retroviral-Mediated Transfection)

This experiment demonstrates the delivery of plasmid DNA encoding the EGFP ribozyme family directly to mammalian cells, thereby, eliminating the use of *E. coli* to amplify the plasmid and increasing the throughput of ribozyme sequences that can be examined. pCEP4 DNAs containing the EGFP ribozyme family were introduced into host cells by calcium-phosphate precipitation (Cochran *et al*. (1985) *Proc. Natl. Acad. Sci.* (USA) 82:19-23, Keck *et al*. (1990) *Cell* 61:801-809). The pCEP4 DNA can be treated with gyrase (Mizuuchi *et al*. (1984) *J. Biol. Chem.* 259:9199-9201; Bates *et al*. (1996) *Biochemistry* 35:1408-1416) to increase the transfection efficiency. In either case, by increasing the amount of transfected DNA from the ligation reaction, more than one ribozyme targeted to a specific sequence can be transfected per cell. This insures that the gene of interest is inactivated and produces an altered phenotype.

### Example 7

### Analysis of Ribozyme Activity

This experiment was designed to demonstrate the inactivation of EGFP expression in target, cells infected with a family of recombinant plasmid vectors that express a family of ribozymes targeted to various regions of the EGFP mRNA. Ribozyme activity is inversely proportional to EGFP expression. The ribozyme hybridizes to and cleaves the EGFP mRNA, thereby, reducing protein expression. EGFP was assayed according to the procedure described in Example 4.

CHO-EGFP cells (Clontech, Palo Alto, CA) were cultured to near confluency 96-well plates in Minimal Essential Media (MEM) supplemented with 5% fetal calf serum (FCS). The family of recombinant plasmids from Example 6 were used to individually and in pools to transfect CHO-EGFP cells. Approximately, 0.1-0.5 micrograms of plasmid DNA was transfected per well to insure that every cell was transfected with at least one plasmid. Mock transfected cell served as controls. Following transfection, the CHO-EGFP cells were incubated for 48-72 hours. EGFP was assayed in Example 4.

### Example 8

### Ribozyme Vector Design with Double-Stranded Oligonucleotides

This experiment was designed to demonstrate the feasibility of novel ribozyme vector production by incorporation of a double-stranded oligonucleotide encoding a ribozyme into a delivery vector followed by site-directed mutagenesis.

Plasmids pGEM-5Zf (-) (Promega) and pGEMEX-1 (Promega) were digested with restriction enzymes *Not*I/*Nsi*I and *Not*I/*Apa*I, respectively, and treated with calf-intestine alkaline phosphatase to dephosphorylate the plasmids. Oligo 1 (5' GGCCGAATTCCT GATGAGGCAGTGATGCCGAAAAGCTTTGCA 3'), SEQ ID NO:23, oligo2 (5' pGGCCGAATT CCTGATGAGGCAGTGATGCCGAAAAGCTTGGCC 3'), SEQ ID NO:24, and oligo3 (5' pAAGCTTT TCGGCATCACTGCCTCATCAGGAATTC 3'), SEQ ID NO:25, were synthesized with a 5' phosphate and obtained from Operon Technologies, Inc. Oligo1 and oligo3 were annealed at a 1:1 molar ratio at room temperature to form a double-stranded DNA molecule with unique *Not*I and *Nsi*I sites at the ends as demonstrated below, while oligo2 and oligo3 were annealed for fifteen minutes at a 1:1 molar ratio at room temperature to form the following double-stranded DNA molecule with unique *Not*I and *Apa*I sites at the ends. Oligo1/3 and oligo2/3 were ligated for one hour at room temperature into *Not*I/*Nsi*I cleaved pGEM and *Not*I/*Apa*I cleaved pGEMEX at a 4:1 molar ratio respectively, with the Epicentre Fast-Link DNA Ligation Kit following the manufacturer's protocol. HB101 competent cells (Life Technologies) were transformed with the ligation reactions by heat shocking at 42°C, plated on LB/Amp plates and incubated at 37°C. Isolated colonies were picked and inoculated into 3 ml. of LB/Amp media and incubated at 37°C overnight. Miniprep plasmid DNA was extracted from the overnight cultures with QIAGEN's QIAprep Spin Miniprep Kit according to the manufacturer's protocol.

Plasmids that contained the proper insert were identified on the basis of a diagnostic restriction enzyme analysis. pGEM-oligo 1/3 was cleaved with *Hind*III which linearized the vector, while pGEMEX-oligo2/3 was cleaved with *Pvu*II which cleaved the vector resulting in the formation of three DNA fragments which were visualized after agarose gel electrophoresis. Plasmids that contained the proper insert were selected for site-directed mutagenesis following Stratagene's QuikChange Site-Directed Mutagenesis Kit. pGEM-oligo1/3 was combined with primers pGEM.Sca (5' TACTCAAGCTATGCAAGTACTT TCGGCATCACTGCCTCATC 3'), SEQ ID NO:26, and pGEM.Pvu (5' ATCACTA GTGCGGCCCAGCTGCTGATGAGGCAGTGATGCCG 3'), SEQ ID NO:27, while pGEMEX-oligo2/3 was combined with primers pGEMEX.Sma (5' CGAGGGATCCG GGCCCCCGGGTTCGGCATCACTGCCTCATC 3'), SEQ ID NO:28, and pGEMEX.Kpn (5' CTTATGCATGCGGCCGGTACCCTGATGAGGCAGTGTGATGCCG 3'), SEQ ID NO:29. The primers were complementary to the plasmid templates except for sequences encoding unique restriction sites that were engineered into the primers and propagated in the polymerase chain reaction (PCR) products. The PCR was performed under the following cycling parameters: 1 cycle at 95°C. for 30 sec.; 18 cycles at 95°C. for 30 sec., 55°C. for 60 sec., 68°C. for 6 min.; and a hold cycle at 4°C. The non-mutated template DNA was then eliminated by digestion with *Dpn*I enzyme. DH5α Max Efficiency competent cells (Life Technologies) were transformed with the mutated PCR products, plated on LB/Amp agar plates and incubated at 37°C. overnight. Isolated colonies were selected; miniprep cultures were grown in LB/Amp at 37°C overnight. Miniprep DNA was extracted from the overnight cultures with QIAGEN's QIAprep Spin Miniprep Kit according to the manufacturer's protocol. pGEM-Sca/Pvu plasmid DNA was cleaved with either PvuII alone or ScaI alone to identify plasmids that contained the proper insert. pGEMEX-Sma/Kpn plasmid DNA was cleaved with *Sma*I and visualized after agarose gel electrophoresis to identify those plasmids that contained an insert. Plasmids with inserts were sequenced to confirm the presence of the mutation. As shown in Fig. 2, pGEM-Sca/Pvu contained the mutated sequence. pGEMEX-Sma/Kpn contained the correctly mutated insert, while the parental plasmid (pGEMEX-oligo2/3), which was not subjected to mutagenesis, contained the wild type sequence.

### Example 9

### Ribozyme Vector Design With Single-Stranded Oligonucleotides

This experiment was designed to demonstrate the feasibility of producing novel ribozyme-vectors by ligating into linearized expression vectors single-stranded oligonucleotides encoding novel ribozymes.

Oligo1 was ligated into *Not*I/*Nsi*I cleaved pGEM and oligo2 was ligated into the *Not*I/*Apa*I cleaved pGEMEX plasmid at a 4:1 molar ratio for one hour at room temperature with the Epicentre Fast-Link DNA Ligation Kit following the manufacturer's protocol. DNA was purified by phenol:chloroform extraction and ethanol precipitation. The vacuum-dried DNA pellet was resuspended with QH₂O. Half of the purified DNA was treated with Klenow so as to fill in the missing complementary strand of the incorporated oligonucleotide; the other half was not treated with Klenow. The sample treated with Klenow was further purified by phenol:chloroform extraction.

PCR was used to determine if oligol and oligo2 were ligated correctly into the vectors. Three microliters of either the Klenow-treated (filled in) template or non-Klenow-treated (not filled in) template were subjected to PCR. pGEM-oligo1 was amplified with either pGEM.for.1 (5' TTGGGCCCGACGTCGCATG 3'), SEQ ID NO:30, or pGEM.for.2 (5' GAATTGGGCCCGACGTCGC 3'), SEQ ID NO:31, combined with pGEM.rev (5' TGTGAGCGGATAACAATTTCACAC 3'), SEQ ID NO:32. pGEMEX-oligo2 was amplified with pGEMEX.for primer (5' GAATACTCAAGCTTATGCATG 3'), SEQ ID NO:33, and pGEMEX.rev primer (5' GAGGTTGTAGAAGTTCCGC 3'), SEQ ID NO:34. The PCR reaction mixtures contained 50 pmoles of each of the forward and reverse primers, 10 mM dNTPs, and 1U ofTaq polymerase in a 50 uL. reaction mixture under the following cycling parameters: 1 cycle at 95°C. for 5 min.; 30 cycles at 95°C. for 30 sec., 55°C. for 30 sec., 68°C. for 1 min.; and a hold cycle at 4°C. Visualization of the PCR products derived from both templates after agarose gel electrophoresis confirmed that oligo1 and oligo2 were properly ligated into the vectors.

Max Efficiency DH5α competent cells (Life Technologies) were transformed with 1 uL. from both samples (filled-in and not filled-in) from each vector by heat shocking at 42°C, plated on LB/Amp plates, and incubated overnight at 37 C. Positive colonies were inoculated into 3 ml. LB/Amp cultures and incubated at 37°C overnight. Plasmid miniprep DNA was isolated with QIAGEN's QIA prep Spin Miniprep Kit and subjected to sequence analysis. As shown in Fig. 3, each plasmid (Klenow treated or not Klenow treated) obtained from the pGEMEX vector contained the properly ligated oligonucleotide in the now double-stranded, circularized ribozyme vector. pGEMEX-oligo2 clones contained the appropriate insert while clones from the parental pGEMEX plasmid did not.

### Example 10

### Ribozvme Vector Design With Triple-Stranded Oligonucleotides

This experiment was designed to demonstrate a novel method for production of ribozyme vectors by ligation of triple-stranded oligonucleotides encoding novel ribozymes into expression vectors.

pGEM was digested with restriction enzymes *Not*I/*Apa*I while pGEMEX was digested with *Not*I/*Nsi*I. The linearized plasmids were treated with calf-intestine alkaline phosphatase to dephosphorylate the plasmids. Oligo4 (5' CATCAGGAATTCGGCCGGCC 3'), SEQ ID NO:35, oligo5 (5' GGCCTGCAAAGCTTTTCGG 3'), SEQ ID NO:36, and oligo6 (5' CATCAGGAATTCGGCCTGCA 3'), SEQ ID NO:37, were treated with T4 polynucleotide kinase to phosphorylate the 5' end of each oligonucleotide and facilitate ligation. Oligo1, oligo4 and oligo5 were annealed for approximately fifteen minutes at a 1:1:1 molar ratio to form the following triple-stranded oligonucleotide. Oligo1, oligo5 and oligo6 were annealed at a 1:1:1 molar ration at room temperature for approximately 15 minutes to form the following triple stranded oligonucleotide. Oligo1/4/5 and oligo 1/5/6 were ligated into *Not*I/*Apa*I cleaved pGEM vector and *Not*I/*Nsi*I cleaved pGEMEX vector, respectively, at either a 5:1 or 6:1 molar ratio using T4 DNA ligase at 400 u/microliter (New England Biolab). After an overnight incubation at 16°C.. the ligation reaction was divided into two equal parts. One part was treated with Klenow to fill in the nicks between the oligonucleotides. The other half of the ligation reaction was not treated with Klenow. The Klenow-treated DNA was purified by phenol:chloroform extraction.

PCR was used to determine if the oligonucleotides were correctly ligated into the vector. Three microliters of either the Klenow-treated template or non-Klenow-treated template were subjected to PCR with pGEM.for.2 and pGEM.rev primers (pGEM-oligo1/4/5 template) or pGEMEX.for and pGEMEX.rev primers (pGENMX-oligo1 /5/6 template) in a PCR reaction mixture containing 50 pmoles of each forward and reverse primer, 10 mM dNTPs, and 1U ofTaq polymerase in a 50 uL. volume under the following parameters: 1 cycle at 95°C. for 5 min.; 30 cycle at 95°C. for 30 sec., 55°C. for 30 sec., 68°C. for 1 min.; and a hold cycle at 4°C. Visualization of the PCR products (5 uL.) derived from pGEMEX-oligo1/5/6, after agarose gel electrophoresis, confirmed that the three oligonucleotides were properly ligated into the vector. The PCR products from the filled-in ligation reactions were not notably different from the non-filled-in ligation reactions. PCR products from pGEM-oligo1/4/5 were not amplified.

Max Efficiency DH5α competent cells (Life Technologies) were transformed with both samples (filled-in and not filled-in) from the pGEMEX-oligo1/5/6 vector by heat shocking at 42°C, plated on LB/Amp plates and incubated overnight at 37°C. Positive colonies (from each sample) were inoculated into 3 ml. of LB/Amp. Plasmid miniprep DNA was isolated from each culture with QIAGEN's QIAprep Spin Miniprep Kit and subjected to sequence analysis. As shown in Fig. 4, each plasmid (Klenow treated or not Klenow treated) from pGEMEX-oligo1/5/6 contained the properly ligated oligonucleotides in the new ribozyme vector.

### Example 11

### Construction of Marker Plasmid

This experiment was designed to disclose the construction of plasmids encoding a gene of interest (EGFP) that can be cloned directly in non-bacterial cells. Sequences encoding green fluorescent protein were ligated into an expression vector. Mammalian cells were transfected with the purified vector and fluorescence was measured as an indication of the level of expression of green fluorescent protein.

The plasmid pLEIN(Clontech) is a Moloney Murine Leukemia Virus derived retroviral vector containing a sequence encoding green fluorescent protein (EGFP) under the control of the 5' viral LTR promoter. 100 µg of pLEIN was digested with 100 units of *Eco*RI restriction enzyme for 3.5 hours at 37°C. The digested DNA was run on a 1% agarose gel to confirm that the digestion reaction had gone to completion. Distinct bands for the vector and the insert (EGFP) were identified. The DNA was purified by phenol chloroform extraction. The purified DNA was incubated with ligase in the presence of ATP at 16°C overnight. Visualization of the ligation reaction mixture on a 1% agarose gel confirmed that the vector and insert had been religated.

### Example 12

### Non-Bacterial Cloning of Marker Plasmid

This experiment was designed to demonstrate that non-bacterial cloning of plasmid DNA containing a gene of interest (EGFP) was sufficient for expression of the gene. Nxa cells were plated at 8x10⁴ cells per well in a 24 well plate one day prior to the transfection. Two hours prior to the transfection, the medium was aspirated from each well and replaced with 1.6 mL of fresh medium. Using the calcium phosphate co-precipitation method, one microgram or five micrograms of supercoiled DNA, cut DNA (not ligated), or religated DNA was transfected into cells of the respective wells. Fresh medium was added to each well three hours after transfection. Cells were incubated at 37°C and 5% CO₂ for 48 hours at which point the cells were observed for expression of green fluorescent protein under a fluorescence microscope. Cells that were transfected with cleaved unligated DNA showed only a low background level of EGFP expression (green fluorescence). Cells that were transfected with one microgram of religated plasmid DNA showed good expression of GFP while cells that were transfected with five micrograms of religated plasmid demonstrated an even brighter green fluorescence. As a positive control, cells that were transfected with either one or five micrograms of supercoiled, uncut DNA showed very bright green fluorescence. These results demonstrated that direct cloning of plasmid DNA in non-bacterial cells was sufficient for expression of green fluorescent protein.

The subject invention discloses methods and compositions for the high-throughput delivery and intracellular expression of nucleic acids to determine the function of a product(s) encoded by a target nucleic acid of interest. Methods are described for the construction of vectors that express nucleic acids that encode either polypeptides, fusion polypeptides, antisense RNAs or ribozymes; the ligation of the nucleic acids-to-be-expressed into either retrovirus vectors or plasmid vectors: the packaging of the recombinant vector into retrovirus particles: the expression of the encoded family from cells either infected with the retrovirus particles or cells directly transfected without a bacterial amplification step with the recombinant plasmid expression vectors. The results demonstrate that a family of oligonucleotide DNA expressing a ribozyme from either recombinant retrovirus or recombinant plasmid expression vectors inactivate a gene of interest to produce an altered cellular phenotype.

All publications and patent applications mentioned in this specification are indicative of the level of skill of those skilled in the art to which this invention pertains.

The invention now having been fully described, it will be apparent to one of ordinary skill in the art that many changes and modifications can be made thereto without departing from the scope of the appended Claims.

## Claims

1. A method of assigning a function to a product encoded by a nucleotide sequence of a sample nucleic acid, the method comprising:
a) without any intervening bacterial cloning steps, delivering into and amplifying and expressing one or more members of an oligonucleotide family as individual transcription products in a plurality of recombinant non-bacterial host cells, comprising a target nucleic acid molecule that comprises the nucleotide sequence of the sample nucleic acid, wherein:
the coding sequences for each individual transcription product encodes an antisense nucleic acid that, when expressed as RNA, binds to mRNA transcribed from the target nucleic acid molecule that comprises the nucleotide sequence of the sample nucleic acid; and
expression of one or more of the individual transcription products prevents production of a product of the mRNA;
b) in the resulting host cells, analyzing phenotypic changes to thereby identify one or more altered function(s), and;
c) based upon the altered function(s), assigning a function to the product encoded by the nucleotide sequence of the sample nucleic acid.

2. The method of claim 1, wherein the function assigned to the product is a physiological function, an enzyme activity, a protein synthesis, expression of a biological factor, or a regulatory effector function.

3. The method of claim 1 wherein said phenotypic change is monitored directly.

4. The method of claim 1 or claim 2, wherein each member of the family of oligonucleotides encodes:
a catalytic domain that, when expressed as RNA, cleaves an mRNA sequence transcribed from the target nucleic acid molecule; and
a binding sequence flanking the catalytic domain for binding the RNA to the mRNA.

5. The method of claim 1, wherein the one or more members of an oligonucleotide family are introduced into expression vectors, which are introduced into the host cells, wherein the expression vectors comprise:
double-stranded DNA, comprising:
a sense strand and an antisense strand, wherein the sense strand codes for a nucleic acid that, when expressed as RNA, binds to an mRNA sequence transcribed from the target nucleic acid sequence so that expression of a product from the target nucleic acid is inhibited; and
means for determining directionality of expression, wherein the product is associated with at least one phenotypic property of a host cell containing the mRNA sequence; and wherein the expression vector is expressed in non-bacterial host cells.

6. The method of claim 5, wherein the RNA comprises:
a catalytic domain that cleaves an mRNA sequence transcribed from a target nucleic acid; and
binding sequences flanking the catalytic domain for binding the RNA to the mRNA, and/or wherein the means for determining directionality of expression comprises a different non blunt-ended restriction enzyme site at each end of said double-stranded DNA.

7. The method of claim 6, wherein the double-stranded DNA is formed by contacting a first oligonucleotide with a complementary second oligonucleotide, and/or wherein the non blunt-ended restriction enzyme site is complementary to an end of the expression vector.

8. The method of any one of claims 5 to 7, wherein said expression vector is formed by: (a) contacting a double-stranded oligonucleotide with an expression vector; or (b) by contacting a single-stranded oligonucleotide with said expression vector; or (c) contacting a triple-stranded oligonucleotide with an expression vector.

9. The method of any one of claims 1-8, wherein the expression vector is a plasmid or a virus that is expressed in non-bacterial host cells.

10. The method of claim 9, wherein the virus is a retrovirus or an adeno-associated virus.

11. The method of any of claims 1-8, wherein the expression vector is transfected directly into mammalian cells.

12. The method of any one of claim 1-11, wherein the sample nucleic acid is genomic DNA, cDNA, an expressed sequence tag (EST) or RNA.

13. The method of claim 6, wherein the family contains between 3 and 20 members.

14. The method of claim 6, wherein each member of the family is designed to inhibit the production of a product of the target nucleic acid molecule.

15. The method of any of claims 1-14 that is performed in a high throughput format.

16. The method of any of claims 1-15 that is performed in a high throughput format, whereby a plurality of different target nucleic acid molecules and/or sample nucleotide sequences are assessed.

17. A method for expressing a family of nucleic acids encoded in a plasmid expression vector in mammalian host cells without an intervening bacterial cloning step, comprising:
preparing plasmid expression vectors that contain a family of nucleic acids by introducing the nucleic acid family into the vectors;
delivering the resulting plasmid expression vectors that contain the family of nucleic acids into the mammalian host cells; and
amplifying the plasmid expression vectors in the host cells, wherein the each nucleic acid in the family comprises:
double-stranded DNA comprising a sense strand and an antisense strand; the sense strand encodes a catalytic domain that, when expressed as RNA, cleaves an mRNA sequence transcribed from a target nucleic acid, and encodes binding sequences flanking the catalytic domain for binding the RNA to the mRNA;
and wherein a means for determining directionality of expression is included in the double-stranded DNA, whereby the nucleic acids in the plasmid expression vectors are expressed in the host cells.

18. The method according to Claim 17, wherein the plasmid expression vector is a retrovirus expression vector.

19. The method according to Claim 17, wherein the plasmid expression vector is an adeno-associated virus expression vector.

20. The method according to Claim 17, wherein the plasmid expression vector is contacted with gyrase.

## Patentansprüche

1. Verfahren zur Zuordnung einer Funktion eines Produkts, welches durch eine Nukleotidsequenz einer Probennukleinsäure kodiert ist, wobei das Verfahren umfasst:
a) Einbringen und Amplifizieren und Exprimieren eines oder mehrerer Mitglieder einer Oligonukleotid-Familie als individuelle Transkriptionsprodukte ohne jegliche zwischenzeitliche bakterielle Klonierungsschritte in mehrere rekombinante nicht-bakterielle Wirtszellen, welche ein die Nukleotidsequenz der Probennukleinsäure umfassendes Zielnukleinsäure-Molekül umfassen, wobei
die für jedes individuelle Transkriptionsprodukt kodierenden Sequenzen eine Antisense-Nukleinsäure kodieren, welche, wenn als RNA exprimiert, an mRNA bindet, die durch Transkription des Zielnukleinsäure-Moleküls erhalten wird, welches die Nukleotidsequenz der Probennukleinsäure umfasst, und
die Expression des einen oder mehrerer individueller Transkriptionsprodukte(s) die Produktion eines Produktes der mRNA verhindert,
b) Analysieren phänotypischer Veränderungen in den resultierenden Wirtszellen zur Identifizierung einer veränderten oder mehrerer veränderter Funktion(en), und
c) basierend auf der/den veränderten Funktion(en) Zuordnen einer Funktion des Produktes, welches durch die Nukleotidsequenz der Probennukleinsäure kodiert ist.

2. Verfahren nach Anspruch 1, wobei die dem Produkt zugeordnete Funktion eine physiologische Funktion, eine Enzymaktivität, eine Proteinsynthese, die Expression eines biologischen Faktors, oder eine regulatorische Effektorfunktion ist.

3. Verfahren nach Anspruch 1, wobei die phänotypische Veränderung direkt beobachtet wird.

4. Verfahren nach Anspruch 1 oder 2, wobei jedes Mitglied der Oligonukleotid-Familie
eine katalytische Domäne, welche, wenn als RNA exprimiert, eine mRNA-Sequenz spaltet, die durch Transkription des Zielnukleinsäure-Moleküls erhalten wird, und
eine Bindungssequenz, welche die katalytische Domäne für eine Bindung der RNA an die mRNA flankiert, kodiert.

5. Verfahren nach Anspruch 1, wobei das eine oder die mehreren Mitglied(er) einer Oligonukleotid-Familie in Expressionsvektoren eingeführt werden, welche in die Wirtszellen eingeführt werden, wobei die Expressionsvektoren umfassen:
Doppelstrang-DNA, umfassend einen Sense-Strang und einen Antisense-Strang, wobei der Sense-Strang für eine Nukleinsäure kodiert, welche, wenn als RNA exprimiert, an eine mRNA-Sequenz bindet, die durch Transkription der Zielnukleinsäure-Sequenz erhalten wird, so dass die Expression eines Produktes der Zielnukleinsäure inhibiert wird, und
Mittel zur Bestimmung der Direktionalität der Expression, wobei das Produkt mit mindestens einer phänotypischen Eigenschaft einer die mRNA-Sequenz enthaltenden Wirtszelle assoziiert ist,
und wobei der Expressionsvektor in nicht-bakteriellen Wirtszellen exprimiert wird.

6. Verfahren nach Anspuch 5, wobei die RNA
eine katalytische Domäne, welche eine mRNA-Sequenz spaltet, die durch Transkription einer Zielnukleinsäure erhalten wird, und
Bindungssequenzen, welche die katalytische Domäne für eine Bindung der RNA an die mRNA flankieren,
umfasst, und/oder wobei das Mittel zur Bestimmung der Direktionalität der Expression an jedem Ende der Doppelstrang-DNA eine unterschiedliche Restriktionsenzym-Schnittstelle ohne glattes Ende umfasst.

7. Verfahren nach Anspruch 6, wobei die Doppelstrang-DNA durch Inkontaktbringen eines ersten Oligonukleotids mit einem komplementären zweiten Oligonukleotid gebildet wird, und/oder wobei die Restriktionsenzym-Schnittstelle kein glattes Ende besitzt und komplementär zu einem Ende des Expressionsvektors ist.

8. Verfahren nach einem der Ansprüche 5 bis 7, wobei der Expressionsvektor gebildet wird durch:
a) Inkontaktbringen eines doppelsträngigen Oligonukleotids mit einem Expressionsvektor, oder
b) Inkontaktbringen eines einzelsträngigen Oligonukleotids mit dem Expressionsvektor, oder
c) Inkontaktbringen eines tripelsträngigen Oligonukleotids mit einem Expressionsvektor.

9. Verfahren nach einem der Ansprüche 1 bis 8, wobei der Expressionsvektor ein Plasmid oder ein Virus ist, welches in nicht-bakteriellen Wirtszellen exprimiert wird.

10. Verfahren nach Anspruch 9, wobei das Virus ein Retrovirus oder ein Adenoassoziiertes Virus ist.

11. Verfahren nach einem der Ansprüche 1 bis 8, wobei der Expressionsvektor direkt in Säugetierzellen transfiziert wird.

12. Verfahren nach einem der Ansprüche 1 bis 11, wobei die Probennukleinsäure eine genomische DNA, cDNA, ein exprimiertes Sequenz Tag (EST) oder RNA ist.

13. Verfahren nach Anspruch 6, wobei die Familie zwischen 3 und 20 Mitglieder enthält.

14. Verfahren nach Anspruch 6, wobei jedes Mitglied der Familie vorgesehen ist, die Produktion eines Produktes des Zielnukleinsäure-Moleküls zu inhibieren.

15. Verfahren nach einem der Ansprüche 1 bis 14, welches in einem Hochdurchsatz-Format ausgeführt wird.

16. Verfahren nach einem der Ansprüche 1 bis 15, welches in einem Hochdurchsatz-Format ausgeführt wird, wobei mehrere unterschiedliche Zielnukleinsäure-Moleküle und/oder Probennukleotid-Sequenzen bewertet werden.

17. Verfahren zur Expression einer Familie von Nukleinsäuren, welche in einem Plasmid-Expressionsvektor in Säugetier-Wirtszellen ohne einen zwischenzeitlichen bakteriellen Klonierungsschritt kodiert werden, umfassend:
Herstellen von Plasmid-Expressionsvektoren, welche durch Einführung der Nukleinsäure-Familie in die Vektoren eine Familie von Nukleinsäuren enthalten,
Einbringen der resultierenden Plasmid-Expressionsvektoren, welche die Nukleinsäure-Familie enthalten, in die Säugetier-Wirtszellen, und
Amplifizieren der Plasmid-Expressionsvektoren in den Wirtszellen, wobei jede Nukleinsäure der Familie Doppelstrang-DNA, umfassend einen Sense-Strang und einen Antisense-Strang, umfasst, wobei der Sense-Strang eine katalytische Domäne kodiert, welche, wenn als RNA exprimiert, eine mRNA-Sequenz spaltet, die durch Transkription einer Zielnukleinsäure erhalten wird, und Bindungssequenzen kodiert, welche die katalytische Domäne für eine Bindung der RNA an die mRNA flankieren,
und wobei die Doppelstrang-DNA ein Mittel zur Bestimmung der Direktionalität der Expression enthält, wobei die Nukleinsäuren in den Plasmid-Expressionsvektoren in den Wirtszellen exprimiert werden.

18. Verfahren nach Anspruch 17, wobei der Plasmid-Expressionsvektor ein retroviraler Expressionsvektor ist.

19. Verfahren nach Anspruch 17, wobei der Plasmid-Expressionsvektor ein Expressionsvektor auf Basis eines Adeno-assoziierten Virus ist.

20. Verfahren nach Anspruch 17, wobei der Plasmid-Expressionsvektor mit Gyrase in Kontakt gebracht wird.

## Revendications

1. Méthode pour attribuer une fonction à un produit codé par une séquence de nucléotides de l'acide nucléique d'un échantillon, méthode comprenant les étapes consistant :
a) sans aucune étape de clonage bactérien intermédiaire, à délivrer, et à amplifier et exprimer un ou plusieurs membres d'une famille d'oligonucléotides sous forme de produits de transcription distincts, dans une pluralité de cellules non bactériennes recombinantes, comprenant une molécule d'acide nucléique cible qui comprend la séquence de nucléotides de l'acide nucléique de l'échantillon, où :
la séquence codant pour chaque produit de transcription distinct code pour un acide nucléique anti-sens qui, lorsqu'il est exprimé sous forme d'un ARN, se lie à l'ARNm transcrit à partir de la molécule d'acide nucléique cible qui comprend la séquence de nucléotides de l'acide nucléique de l'échantillon ; et
l'expression d'un ou plusieurs des produits de transcription distincts empêche la production d'un produit de l'ARNm ;
b) dans les cellules hôtes résultantes, à analyser les modifications phénotypiques pour identifier ainsi une ou plusieurs fonctions modifiées, et ;
c) sur la base de la ou des fonctions modifiées, à attribuer une fonction au produit codé par la séquence de nucléotides de l'acide nucléique de l'échantillon.

2. Méthode suivant la revendication 1, dans laquelle la fonction attribuée au produit est une fonction physiologique, une activité enzymatique, un synthèse de protéines, l'expression d'un facteur biologique ou une fonction d'effecteur de régulation.

3. Méthode suivant la revendication 1, dans laquelle ladite modification phénotypique est contrôlée directement.

4. Méthode suivant la revendication 1 ou la revendication 2, dans laquelle chaque membre de la famille d'oligonucléotides code pour :
un domaine catalytique qui, lorsqu'il est exprimé sous forme d'un ARN, clive une séquence d'ARNm transcrite à partir de la molécule d'acide nucléique cible ; et
une séquence de liaison adjacente au domaine catalytique pour la liaison de l'ARN à l'ARNm.

5. Méthode suivant la revendication 1, dans laquelle le ou les membres d'une famille d'oligonucléotides sont introduits dans des vecteurs d'expression, qui ont été introduits dans les cellules hôtes, où les vecteurs d'expression comprennent :
un ADN bicaténaire, comprenant :
un brin sens et un brin anti-sens, le brin sens codant pour un acide nucléique qui, lorsqu'il est exprimé sous forme d'un ARN, se lie à une séquence d'ARNm transcrite à partir de la séquence d'acide nucléique cible de telle sorte que l'expression d'un produit à partir de l'acide nucléique cible soit inhibée ; et
un moyen pour déterminer la directionalité de l'expression, où le produit est associé à au moins une propriété phénotypique d'une cellule hôte contenant la séquence d'ARNm ; et où le vecteur d'expression est exprimé dans des cellules hôtes non bactériennes.

6. Méthode suivant la revendication 5, dans laquelle l'ARN comprend :
un domaine catalytique qui clive une séquence d'ARNm transcrite à partir d'un acide nucléique cible ; et
des séquences de liaison adjacentes au domaine catalytique pour la liaison de l'ARN à l'ARNm, et/ou dans laquelle le moyen pour déterminer la directionalité de l'expression comprend un site d'enzyme de restriction différent, sans extrémité franche, à chaque extrémité dudit ADN bicaténaire.

7. Méthode suivant la revendication 6, dans laquelle l'ADN bicaténaire est formé en mettant en contact un premier oligonucléotide avec un second oligonucléotide complémentaire, et/ou dans laquelle le site d'enzyme de restriction non à extrémités franches est complémentaire d'une extrémité du vecteur d'expression.

8. Méthode suivant l'une quelconque des revendications 5 à 7, dans laquelle ledit vecteur d'expression est formé : (a) en mettant en contact un oligonucléotide bicaténaire avec un vecteur d'expression ; ou (b) en mettant en contact un oligonucléotide monocaténaire avec ledit vecteur d'expression ; ou bien (c) en mettant en contact un oligonucléotide à trois brins avec un vecteur d'expression.

9. Méthode suivant l'une quelconque des revendications 1 à 8, dans laquelle le vecteur d'expression est un plasmide ou un virus qui est exprimé dans des cellules hôtes non bactériennes.

10. Méthode suivant la revendication 9, dans laquelle le virus est un rétrovirus ou un virus adéno-associé.

11. Méthode suivant l'une quelconque des revendications 1 à 8, dans laquelle le vecteur d'expression est transfecté directement dans des cellules de mammifère.

12. Méthode suivant l'une quelconque des revendications 1 à 11, dans laquelle l'acide nucléique de l'échantillon est un ADN génomique, un ADNc, un marqueur de séquence exprimé (EST) ou un ARN.

13. Méthode suivant la revendication 6, dans laquelle la famille contient 3 à 20 membres.

14. Méthode suivant la revendication 6, dans laquelle chaque membre de la famille est conçu pour inhiber la production d'un produit de la molécule d'acide nucléique cible.

15. Méthode suivant l'une quelconque des revendications 1 à 14, qui est mise en oeuvre dans un format à haut débit.

16. Méthode suivant l'une quelconque des revendications 1 à 15, qui est mise en oeuvre dans un format à haut débit, ce qui permet d'évaluer une pluralité de molécules d'acide nucléique cibles différentes et/ou des séquences de nucléotides de l'échantillon.

17. Méthode pour l'expression d'une famille d'acides nucléiques codés dans un vecteur d'expression consistant en un plasmide dans des cellules hôtes de mammifère sans étape de clonage bactérien intermédiaire, comprenant les étapes consistant :
à préparer des vecteurs d'expression consistant en plasmides, qui contiennent une famille d'acides nucléiques en introduisant la famille d'acides nucléiques dans les vecteurs ;
à délivrer les vecteurs d'expression consistant en plasmides résultants qui contiennent la famille d'acides nucléiques aux cellules hôtes de mammifères ; et
à amplifier les vecteurs d'expression consistant en plasmides dans les cellules hôtes, où chaque acide nucléique dans la famille comprend :
un ADN bicaténaire comprenant un brin sens et un brin anti-sens ; le brin sens code pour un domaine catalytique qui, lorsqu'il est exprimé sous forme d'un ARN, clive une séquence d'ARNm transcrite à partir d'un acide nucléique cible, et code pour des séquences de liaison adjacentes au domaine catalytique pour la liaison de l'ARN à l'ARNm ; et
où un moyen pour déterminer la directionalité de l'expression est incorporé à l'ADN bicaténaire, ce qui fait que les acides nucléiques présents dans les vecteurs d'expression consistant en plasmides sont exprimés dans les cellules hôtes.

18. Méthode suivant la revendication 17, dans laquelle le vecteur d'expression consistant en un plasmide est un vecteur d'expression rétroviral.

19. Méthode suivant la revendication 17, dans laquelle le vecteur d'expression consistant en un plasmide est un vecteur d'expression viral adéno-associé.

20. Méthode suivant la revendication 17, dans laquelle le vecteur d'expression consistant en un plasmide est mis en contact avec un gyrase.


### SEQUENCE LISTING

(1) GENERAL INFORMATION:
   (i) APPLICANT: Keck, James G; Molony, Jocelyn M; Kuo, Sophia S
   (ii) TITLE OF INVENTION: Non-Bacterial Cloning in Delivery and Expression of Nucleic Acids
   (iii) NUMBER OF SEQUENCES: 46
   (iv) CORRESPONDENCE ADDRESS :
      (A) ADDRESSEE: Rae-Venter Law Group, P.C.
      (B) STREET: P.O. Box 60039
      (C) CITY: Palo Alto
      (D) STATE: California
      (E) COUNTRY: USA
      (F) ZIP: 94306-0039
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30
   (vi) CURRENT APPLICATION DATA:
      (A) APPLICATION NUMBER:
      (B) FILING DATE: 18-DEC-1998
      (C) CLASSIFICATION:
   (viii) ATTORNEY/AGENT INFORMATION:
      (A) NAME: Rae-Venter, Barbara
      (B) REGISTRATION NUMBER: 32,750
      (C) REFERENCE/DOCKET NUMBER: STBI.001.01US
   (ix) TELECOMMUNICATION INFORMATION:
      (A) TELEPHONE: (650) 328-4400
      (B) TELEFAX: (650) 328-4477
(2) INFORMATION FOR SEQ ID NO:1:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
   (xi) SEQUENCE DESCRIPTION: SEQ ID NO:1:
   CCAGCTCCTG ATGAGTCCGT GAGGACGAAA CCAGGA 36
(2) INFORMATION FOR SEQ ID NO:2:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:2:
   GGCCGTTCTG ATGAGTCCGT GAGGACGAAA CGTCGC 36
(2) INFORMATION FOR SEQ ID NO:3:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:3:
   CTCGCCGCTG ATGAGTCCGT GAGGACGAAA CACGCT 36
(2) INFORMATION FOR SEQ ID NO:4:
   (i) SEQUENCE CHARACTERISTICS :
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:4:
   GCAGATGCTG ATGAGTCCGT GAGGACGAAA CTTCAG 36
(2) INFORMATION FOR SEQ ID NO:5:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:5:
   TGGTCACCTG ATGAGTCCGT GAGGACGAAA GGGTGG 36
(2) INFORMATION FOR SEQ ID NO:6:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:6:
   AGCGGCTCTG ATGAGTCCGT GAGGACGAAA GGCACT 36
(2) INFORMATION FOR SEQ ID NO:7:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:7:
   CATGGCGCTG ATGAGTCCGT GAGGACGAAA CTTGAA 36
(2) INFORMATION FOR SEQ ID NO:8:
   (i) SEQUENCE CHARACTERISTICS:
      (A) LENGTH: 36 base pairs
      (B) TYPE: nucleic acid
      (C) STRANDEDNESS: double
      (D) TOPOLOGY : linear
   (ii) MOLECULE TYPE: other nucleic acid
      (A) DESCRIPTION: /desc = "Synthetic DNA"
   (xi) SEQUENCE DESCRIPTION: SEQ ID NO:8:
   GCTCCTGCTG ATGAGTCCGT GAGGACGAAA CGTAGC 36
(2) INFORMATION FOR SEQ ID NO:9:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:9:
   CGTCCTTCTG ATGAGTCCGT GAGGACGAAA AGAAGA 36
(2) INFORMATION FOR SEQ ID NO:10:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:10:
   CGCCCTCCTG ATGAGTCCGT GAGGACGAAA ACTTCA 36
(2) INFORMATION FOR SEQ ID NO:11:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:11:
   TGCGGTTCTG ATGAGTCCGT GAGGACGAAA CCAGGG 36
(2) INFORMATION FOR SEQ ID NO:12:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:12:
   CCTCCTTCTG ATGAGTCCGT GAGGACGAAA AGTCGA 36
(2) INFORMATION FOR SEQ ID NO:13:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:13:
   GTAGTTGCTG ATGAGTCCGT GAGGACGAAA CTCCAG 36
(2) INFORMATION FOR SEQ ID NO:14:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:14:
   TGATATACTG ATGAGTCCGT GAGGACGAAA CGTTGT 36
(2) INFORMATION FOR SEQ ID NO:15:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:15:
   GGATCTTCTG ATGAGTCCGT GAGGACGAAA AGTTCA 36
(2) INFORMATION FOR SEQ ID NO:16:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:16:
   GGTCGGCCTG ATGAGTCCGT GAGGACGAAA GCTGCA 36
(2) INFORMATION FOR SEQ ID NO:17:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:17:
   GCAGCAGCTG ATGAGTCCGT GAGGACGAAA CGGGGC 36
(2) INFORMATION FOR SEQ ID NO:18:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:18:
   CAGGGCGCTG ATGAGTCCGT GAGGACGAAA CTGGGT 36
(2) INFORMATION FOR SEQ ID NO:19:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:19:
   CCAGCAGCTG ATGAGTCCGT GAGGACGAAA CCATGT 36
(2) INFORMATION FOR SEQ ID NO:20:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:20:
   CCATGCCCTG ATGAGTCCGT GAGGACGAAA GAGTGA 36
(2) INFORMATION FOR SEQ ID NO:21:
   (i) SEQUENCE CHARACTERISTICS:
      (A) LENGTH: 43 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:21:
   AGCTTCCAGC TCCTGATGAG TCCGTGAGGA CGAAACCAGG AAT 43
(2) INFORMATION FOR SEQ ID NO:22:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:22:
   CGATTCCTGG TTTCGTCCTC ACGGACTCAT CAGGAGCTGG A 41
(2) INFORMATION FOR SEQ ID NO:23:
   (i) SEQUENCE CHARACTERISTICS:
      (A) LENGTH: 42 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:23:
   GGCCGAATTC CTGATGAGGC AGTGATGCCG AAAAGCTTTG CA 42
(2) INFORMATION FOR SEQ ID NO:24:
   (i) SEQUENCE CHARACTERISTICS:
      (A) LENGTH: 42 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:24:
   GGCCGAATTC CTGATGAGGC AGTGATGCCG AAAAGCTTGG CC 42
(2) INFORMATION FOR SEQ ID NO:25:
   (i) SEQUENCE CHARACTERISTICS:
      (A) LENGTH: 34 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:25:
   AAGCTTTTCG GCATCACTGC CTCATCAGGA ATTC 34
(2) INFORMATION FOR SEQ ID NO:26:
   (i) SEQUENCE CHARACTERISTICS:
      (A) LENGTH: 41 base pairs
      (B) TYPE: nucleic acid.
      (C) STRANDEDNESS : double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:26:
   TACTCAAGCT ATGCAAGTAC TTTCGGCATC ACTGCCTCAT C 41
(2) INFORMATION FOR SEQ ID NO:27:
   (i) SEQUENCE CHARACTERISTICS:
      (A) LENGTH: 41 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:27:
   ATCACTAGTG CGGCCCAGCT GCTGATGAGG CAGTGATGCC G 41
(2) INFORMATION FOR SEQ ID NO:28:
   (i) SEQUENCE CHARACTERISTICS:
      (A) LENGTH: 41 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:28:
   CGAGGGATCC GGGCCCCCGG GTTCGGCATC ACTGCCTCAT C 41
(2) INFORMATION FOR SEQ ID NO:29:
   (i) SEQUENCE CHARACTERISTICS :
      (A) LENGTH: 43 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:29:
   CTTATGCATG CGGCCGGTAC CCTGATGAGG CAGTGTGATG CCG 43
(2) INFORMATION FOR SEQ ID NO:30:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:30:
   TTGGGCCCGA CGTCGCATG 19
(2) INFORMATION FOR SEQ ID NO:31:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:31:
   GAATTGGGCC CGACGTCGC 19
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:32:
   TGTGAGCGGA TAACAATTTC ACAC 24
(2) INFORMATION FOR SEQ ID NO:33:
   (i) SEQUENCE CHARACTERISTICS:
      (A) LENGTH: 21 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:33:
   GAATACTCAA GCTTATGCAT G 21
(2) INFORMATION FOR SEQ ID NO:34:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:34:
   GAGGTTGTAG AAGTTCCGC 19
(2) INFORMATION FOR SEQ ID NO:35:
   (i) SEQUENCE CHARACTERISTICS:
      (A) LENGTH: 20 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:35:
   CATCAGGAAT TCGGCCGGCC 20
(2) INFORMATION FOR SEQ ID NO:36:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:36:
   GGCCTGCAAA GCTTTTCGG 19
(2) INFORMATION FOR SEQ ID NO:37:
   (i) SEQUENCE CHARACTERISTICS:
      (A) LENGTH: 20 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Synthetic DNA"
   (xi) SEQUENCE DESCRIPTION: SEQ ID NO:37:
   CATCAGGAAT TCGGCCTGCA 20
(2) INFORMATION FOR SEQ ID NO:38:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:38:
   AGTACTTTCG GCATCACTGC CTCATCAGCA GCTGGG 36
(2) INFORMATION FOR SEQ ID NO:39:
   (i) SEQUENCE CHARACTERISTICS:
      (A) LENGTH: 36 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:39:
   AAGCTTTTCG GCATCACTGC CTCATCAGGA ATTCGG 36
(2) INFORMATION FOR SEQ ID NO:40:
   (i) SEQUENCE CHARACTERISTICS:
      (A) LENGTH: 42 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:40:
   GCCAGTACCG ATGGAGGCAG TGATGCCGAA CCCGGGGGCC CG 42
(2) INFORMATION FOR SEQ ID NO:41:
   (i) SEQUENCE CHARACTERISTICS:
      (A) LENGTH: 42 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:41:
   CCGAATTCCT GATGAGGCAG TGATGCCGAA AAGCTTGGCC CG 42
(2) INFORMATION FOR SEQ ID NO:42:
   (i) SEQUENCE CHARACTERISTICS:
      (A) LENGTH: 51 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:42:
   CGGGCCAAGC TTTTCGGCAT CACTGCCTCA TCAGGAATTC GGCCGCATGC A 51
(2) INFORMATION FOR SEQ ID NO:43:
   (i) SEQUENCE CHARACTERISTICS:
      (A) LENGTH: 27 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:43:
   CGGGCCCTCT AGATGCGGCC GCATGCA 27
(2) INFORMATION FOR SEQ ID NO:44:
   (i) SEQUENCE CHARACTERISTICS:
      (A) LENGTH: 57 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:44:
   GGCCTGCAAA GCTTTTCGGC ATCACTGCCT CATCAGGAAT TCGGCCTGCA TAAGCTT 57
(2) INFORMATION FOR SEQ ID NO:45:
   (i) SEQUENCE CHARACTERISTICS:
      (A) LENGTH: 56 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:45:
   GGCTGCAAAG CTTTTCGGCA TCACTGCCTC ATCAGGAATT CGGCCTGCAT AAGCTT 56
(2) INFORMATION FOR SEQ ID NO:46:
   (i) SEQUENCE CHARACTERISTICS:
      (A) LENGTH: 18 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "Plasmid DNA"
   (xi) SEQUENCE DESCRIPTION: SEQ ID NO:46:
   GGCCGCATGC ATAAGCTT 18